# EUROPÄISCHE PATENTANMELDUNG

(11) **EP 1 870 407 A1**
(43) Veröffentlichungstag der Anmeldung: **26.12.2007**
(21) Anmeldenummer: 06012791.7
(22) Anmeldetag: 22.06.2006
(51) Int. Cl.: C07D 317/38

(54) **Verfahren zur Herstellung von Glycerincarbonat-Estern (I)**

(71) Anmelder: Cognis Oleochemicals GmbH, 40589 Düsseldorf (DE)
(72) Erfinder: Westfechtel, Alfred, Dr., 40724 Hilden (DE)
(74) Vertreter: Reinhardt, Jürgen

(57) **Zusammenfassung**

Vorgeschlagen wird ein Verfahren zur Herstellung von Glycerincarbonat-Estern der Formel (I) 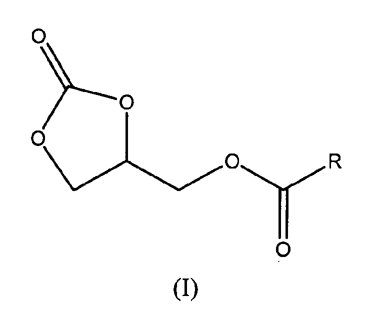 worin R Wasserstoff oder einen Alkyl- oder Alkenylrest mit 1 bis 3 C-Atomen bedeutet, der geradkettig oder verzweigt sein kann, dadurch gekennzeichnet, dass man in Gegenwart eines Umesterungs-Katalysators eine Verbindung der Formel (II) 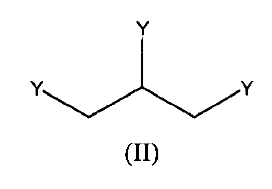 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat umsetzt, wobei die im Zuge der Umesterung gebildete Verbindung CH₃-O-CO-Z oder CH₃-CH₂-O-CO-Z kontinuierlich aus dem Reaktionsgemisch entfernt wird.

## Beschreibung

### Gebiet der Erfindung

Die Erfindung betrifft ein Verfahren zur Herstellung von Glycerincarbonat-Estern.

### Stand der Technik

Ester des Glycerincarbonats sind relativ wenig beschrieben. Gemäß Zephirin Mouloungui et al., Eur. J. Lipid Sci. Technol., 103 (2001) 216 - 222 werden die langkettigen Carbonat-Ester durch Umsetzung von Glycerin-Carbonat mit Säurechloriden erhalten. Im Rahmen einer Übersicht zu cyclischen Carbonat-funktionellen Polymeren stellt Dean C. Webster in Progress in Organic Coatings, 47 (2003), 77-86 mehrere Synthesewege von Glycerincarbonatmethacrylat vor, unter anderem die Umesterung von Glycerincarbonat mit Methylmethacrylat. US 3,225,063 beschreibt die Darstellung von Carbonat-Estern mittels cyclischer Anhydride in Form saurer Halbester.

Acrylaten und Methacrylaten als ungesättigten Estern des Glycerincarbonats sind spezielle Publikationen gewidmet. So beschreibt US 2,979,514 die Umsetzung von Säurechloriden und Glycerincarbonat. Dabei wird mit großen Überschüssen an Reagenzien und Lösemitteln bzw. Schleppmitteln gearbeitet. In der DE 10355830 A1 wird ein Verfahren vorgeschlagen, bei dem Metall-Chelat-Katalysatoren für die Umesterung von Glycerincarbonat mit Methylmethacrylat eingesetzt werden. Auch hierbei wird ein hoher Überschuss Acrylat verwandt.

WO 93/09111 A2 offenbart ein Verfahren zur Herstellung von Glycerincarbonat-Estem, wobei Glycerinester mit C₁₋₂₃ Fettsäuren, insbesondere Triglyceride auf Basis der genannten Fettsäuren, in Gegenwart eines Katalysators mit einem Carbonat, insbesondere Dimethylcarbonat oder Diethylcarbonat umgesetzt werden.

### Beschreibung der Erfindung

Aufgabe der vorliegenden Erfindung war es, ein neues Verfahren zur Herstellung von Glycerincarbonat-Estern bereitzustellen.

Die Erfindung betrifft ein Verfahren zur Herstellung von Glycerincarbonat-Estern der Formel (I) 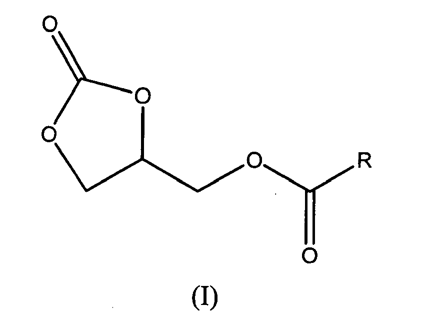 worin R Wasserstoff oder einen Alkyl- oder Alkenylrest mit 1 bis 3 C-Atomen bedeutet, der geradkettig oder verzweigt sein kann, wobei man in Gegenwart eines Umesterungs-Katalysators eine Verbindung der Formel (II) 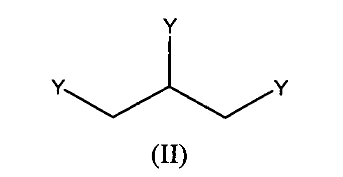 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat umsetzt, wobei die im Zuge der Umesterung gebildete Verbindung CH₃-O-CO-Z oder CH₃-CH₂-O-CO-Z kontinuierlich aus dem Reaktionsgemisch entfernt wird.

Das erfindungsgemäße Verfahren hat mehrere Vorteile: Man geht von preiswertem Rohstoffen aus. Als Nebenprodukt entsteht ein Methylester oder Ethylester einer kurzkettigen Carbonsäure, der neben dem eigentlichen Zielprodukt (I) ein Wertprodukt darstellt (Methylacetat etwa kann als Lösungsmittel verwendet werden). Das erfindungsgemäße Verfahren lässt sich unter schonenden Bedingungen durchführen.

Die Wahl des Umesterungs-Katalysators ist an sich nicht kritisch. Man kann an sich jeden Umesterungskatalysator einsetzen. Besonders geeignete Umesterungskatalysatoren sind Alkoholate wie Natriummethylat, Kaliummethylat oder Hydroxyde wie Natriumhydroxyd und Kaliumhydroxyd sowie Carbonate. Die Katalysatoren werden vorzugsweise in einer Menge von 0,01- 5%, bevorzugt von 0,5 - 1% bezogen auf den Gesamtansatz eingesetzt.

Das kontinuierliche Abdestillieren der bei der Umesterung neben der Zielverbindung (I) entstehenden Verbindung CH₃-O-CO-Z ist ein wesentlicher Parameter des Verfahrens, der sicherstellt, dass die Reaktion unter schonenden Bedingungen durchgeführt werden kann und hellfarbige Produkte liefert.

Die Reaktionstemperatur ist an sich nicht kritisch. Vorzugsweise wird sie zwischen 40 und 150°C durchgeführt. Als besonders bevorzugt hat sich der Bereich zwischen 60 und 110°C erwiesen.

Die Reaktanden werden vorzugsweise in stöchiometrischer Menge und insbesondere in einem leichten Überschuss des Dimethylcarbonat oder Diethylcarbonats miteinander umgesetzt.

Beispiele für besonders geeignete Gruppen Z in den Verbindungen (II) sind: Wasserstoff, Methyl, Ethyl, n-Propyl, i-Propyl sowie -C(CH₃)=CH₂ oder -CH=CH₂.

In einer bevorzugten Ausführungsform hat Z in Formel (II) die Bedeutung einer Methylgruppe.
In einer weiteren bevorzugten Ausführungsform hat Z in Formel (II) die Bedeutung einer Gruppe -C(CH₃)=CH₂ oder -CH=CH₂.

Das erfindungsgemäße Verfahren kann gewünschtenfalls in Gegenwart eines Lösungsmittels durchgeführt werden. Vorzugsweise wird es jedoch in Abwesenheit eines Lösungsmittels durchgeführt.

Gewünschtenfalls kann das Zielprodukt (I) dem Fachmann vertrauten Reinigungsschritten unterworfen werden, um die Reinheit des Produktes zu erhöhen.

Ein weiterer Erfindungsgegenstand ist die Verwendung der Verbindungen (I) als Lösungsmittel oder als hochsiedende Arbeitsflüssigkeiten. Hierbei ist von Vorteil, dass das die Verbindungen (I) bei erhöhten Temperaturen durch Zersetzung CO₂ abgeben können (Flammschutzwirkung). Die Verbindungen (I) können außerdem für folgende Verwendungszwecke eingesetzt werden: Hydrophobe Emulgatoren, halogenfreie Schmiermittelzusätze, z. B. für Treibstoffe.

### Beispiele

### Beispiel 1:

Herstellung von Glycerincarbonat-Acetat (Essigsäureester des Glycerincarbonats)

**Ansatz: Es wurden folgende Mengen eingesetzt:**

| | **mol. Gewicht (g/mol)** | **Menge (mol)** | **Menge (g)** | |
|---|---|---|---|---|
| **Triacetin** | 218 | 9 | 1962 | |
| **Dimethylcarbonat** | 90 | 9,9 | 891 | 10% Überschuss |
| **NaOMe** | 54 | 0,26 | 14,25 (47,Sg von einer 30%igen Lösung) | |
| **HCl (2N)** | 36,5 | 0,26 | 134 | |

Durchführung: Triacetin (= Triessigsäureester des Glycerins), Dimethylcarbonat (DMC) und Natriummethanolat (MeONa) wurden gerührt, dabei entstand eine Dispersion. Unter Stickstoff-Atmosphäre wurde die Temperatur erhöht. Die Destillation begann bei 65-70°C im Sumpf. Die Sumpftemperatur wurde langsam auf 110°C erhöht. Die Destillatmenge von 1412g erreichte relativ genau die theoretische Menge (1446g), die Reinheit des Destillats wurde mittels Refraktion kontrolliert. Das Sumpfprodukt wurde auf 50°C abgekühlt und mit HCl neutralisiert. 70g Wasser wurden hinzugefügt um eine bessere Trennung zu erzielen (Salze wurden gelöst). Die wässerige Phase wurde abgetrennt. Die organische Phase wurde mit 360g Wasser ausgewaschen und bei bei 50°C abgetrennt. Die klare orange organische Phase wurde unter Wasserstrahlvakuum getrocknet.
Das Produkt wurde als klare orange Flüssigkeit erhalten (Dichte=1,34). Die Ausbeute betrug 1500 g (85%). Die Reinheit (GC-Analyse) betrug 98%. Die Identität des Produktes wurde über H-NMR-Spektroskopie abgesichert.

## Patentansprüche

1. Verfahren zur Herstellung von Glycerincarbonat-Estern der Formel (I) 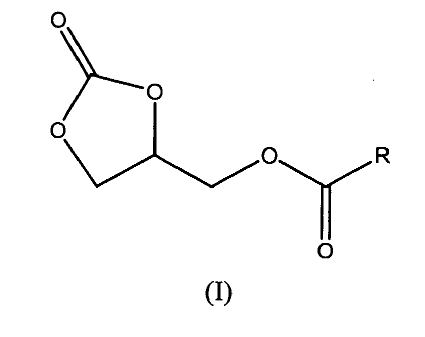 worin R Wasserstoff oder einen Alkyl- oder Alkenylrest mit 1 bis 3 C-Atomen bedeutet, der geradkettig oder verzweigt sein kann, **dadurch gekennzeichnet, dass** man in Gegenwart eines Umesterungs-Katalysators eine Verbindung der Formel (II) 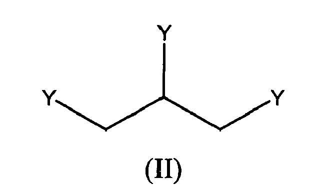 worin Y eine Gruppe -O-CO-Z und Z Wasserstoff oder einen Alkyl- oder Alkenylrest mit 1 bis 3 C-Atomen bedeutet, der geradkettig oder verzweigt sein kann, mit Dimethylcarbonat oder Diethylcarbonat umsetzt, wobei die im Zuge der Umesterung gebildete Verbindung CH₃-O-CO-Z oder CH₃-CH₂-O-CO-Z kontinuierlich aus dem Reaktionsgemisch entfernt wird.

2. Verwendung der Verbindungen (I) als Lösungsmittel, als Arbeitsflüssigkeiten mit Flammschutzwirkung oder Schmiermittelzusatz für Treibstoffe.
